(19)

Europäisches
Patentamt

European
Patent Office

Office européen
des brevets

(11) **EP 3 125 138 A1**

(12) **EUROPEAN PATENT APPLICATION**

(43) Date of publication:
**01.02.2017  Bulletin 2017/05**

(51) Int Cl.:
***G06F 19/00*** *(2011.01)*

(21) Application number: **16075001.4**

(22) Date of filing: **15.01.2016**

| | |
|---|---|
| (84) Designated Contracting States:<br>**AL AT BE BG CH CY CZ DE DK EE ES FI FR GB GR HR HU IE IS IT LI LT LU LV MC MK MT NL NO PL PT RO RS SE SI SK SM TR**<br>Designated Extension States:<br>**BA ME**<br>Designated Validation States:<br>**MA MD** | (72) Inventors:<br>• **Abdulahad, Ayad Kamil**<br>  **2333 BE Leiden (NL)**<br>• **Snijder, Jan Robert**<br>  **2333 BE Leiden (NL)** |
| (30) Priority: **31.07.2015  US 201514814618** | (74) Representative: **Olthoff, Margaretha**<br>**Astellas Pharma Europe BV**<br>**Sylviusweg 62**<br>**2333 BE Leiden (NL)** |
| (71) Applicant: **Astellas Pharma Europe Ltd.**<br>**Chertsey, Surrey KT16 0RS (GB)** | |

(54) **SYSTEM AND METHOD FOR ANALYZING ADVERSE EVENT DATA**

(57)  A non-transitory memory is configured to store adverse event data. The adverse event data describes whether an individual experienced an adverse event as a result of being administered a therapeutic agent on each day during a time period. A processor, operatively connected to the at least one non-transitory memory, is configured to process the adverse event data and generate an output.

EP 3 125 138 A1

**Description**

FIELD OF THE INVENTION

The present invention generally relates to analyzing data describing adverse events experienced by an individual who is being treated with a medication for a condition.

BACKGROUND OF THE INVENTION

During every clinical trial, there is the potential that patients will report treatment-emergent adverse events (TEAEs) (defined as an adverse event that first appears during treatment, which was absent before or which worsens relative to the pre-treatment state), and these must be reported to the study's safety monitoring bodies. Safety data from clinical studies are reported as an absolute list or summary, which does not optimally utilize the detailed information captured in a clinical study database. Current reporting methods provide a limited view of the patient experience, where the safety burden of treatment is only assessed as an overall summary over the study period and the burden of therapy on the patient is not presented. The collective burden of therapy on the individual or treatment group is not evaluated. A novel methodology to report safety during a clinical study from a patient perspective is presented.

A common way of reporting TEAEs is to present the data in a table by showing the number of patients who reported one or more TEAE coded in accordance with the medical dictionary for regulatory activities system organ class in percentage (%) (M. Backonja et al. "NGX-4010, a high-concentration capsaicin patch, for the treatment of postherpetic neuralgia: a randomized double-blind study", Lancet Neurol. 2008 Dec; 7(12):1106-12). Another way of reporting TEAE is providing the number of adverse events in percentage for a specific grade of an TEAE (T.M. Beer et al., "Enzalutamide in Metastatic prostate cancer before chemotherapy"; N Engl J Med, 2014 371(5):424-33). These methods do not allow the identification or differentiation of patients who experience more than one TEAE, either contemporaneously or consecutively. The presence and severity of adverse events on each day of a clinical study is not analyzed. Therefore, there remains a need to develop a method to obtain greater insights of TEAEs reported during clinical studies from the perspective of the patient.

SUMMARY OF THE INVENTION

The present invention is directed to a non-transitory memory configured to store adverse event data. The adverse event data describes whether an individual experienced an adverse event as a result of being administered a therapeutic agent on each day during a time period. A processor, operatively connected to the at least one non-transitory memory, is configured to process the adverse event data and generate an output.

In some embodiments, the adverse event data comprises at least one severity indicator associated with the adverse event experienced on each of the days in the time period. The severity indicator may comprise one of mild severity, moderate severity, and severe severity. The severity indicator is associated with a weight. In some embodiments, the severity indicator for mild severity is 1, the severity indicator for moderate severity is 2, and the severity indicator for severe severity is 3. In some embodiments, if the adverse event is death, the severity indicator is associated with a weight of 5. The severity indicator may comprise a hospitalization indicator, describing whether the adverse event was associated with hospitalization. The severity indicator may comprise a treatment interference indicator, describing whether treatment continued without interruption, was stopped temporarily, or was stopped permanently. Processing the adverse event data comprises associating a weight with each of the one or more severity indicators and summing the weights for each day.

In addition, a serious indicator may be used, describing the level of seriousness associated with the adverse event.

In some embodiments, the non-transitory memory is further configured to store data describing a day, within the time period, on which the individual responded to the therapeutic agent.

In some embodiments, the adverse event data is collected using a questionnaire.

In some embodiments, the output is used to generate a graphical display.

In some embodiments, a plurality of individuals are being administered the therapeutic agent on each of the days during the time period, and the processor is further configured to: determine how many individuals are being administered the therapeutic agent on a given day during the time period; determine how many of the individuals being administered the therapeutic agent on the given day responded to the treatment by the given day; determine a proportion of individuals that responded to the treatment by the given day out of a total number of the plurality of individuals; and determine a median time for response to the treatment for the plurality of individuals.

BRIEF DESCRIPTION OF THE SEVERAL VIEWS OF THE DRAWINGS

Figure 1A shows an exemplary graph that may be generated using the methodology and systems of the present invention, in casu the average burden experienced per day by 6 different patients over a period of 55 days after administration of an anti-convulsant agent.
Figure 1B shows an exemplary graph that may be generated using the methodology and systems of the present invention, in casu the average burden experienced per day by 6 different patients over a period of 55 days after application of a topical TRPV1 agonist.

Figure 2A shows an exemplary graph that may be generated using the methodology and systems of the present invention, in casu the average burden experienced per day over a period of 55 days after administration of an anti-convulsant agent.

Figure 2B shows an exemplary graph that may be generated using the methodology and systems of the present invention, in casu the average burden experienced per day over a period of 55 days after administration of a topical TRPV1 agonist.

Figure 3A shows an exemplary graph that may be generated using the methodology and systems of the present invention, in casu the average burden experienced per day over a period of 85 days after administration of an adrenergic receptor agonist and an antimuscarinic agent respectively.

Figure 3B shows an exemplary graph that may be generated using the methodology and systems of the present invention, in casu the difference in average burden experienced per day over a period of 85 days after administration of an adrenergic receptor agonist and an antimuscarinic agent respectively.

Figure 4A shows an exemplary graph that may be generated using the methodology and systems of the present invention, in casu the average dry mouth burden experienced per day over a period of 85 days after administration of an adrenergic receptor agonist and an antimuscarinic agent respectively.

Figure 4B shows an exemplary graph that may be generated using the methodology and systems of the present invention, in casu the difference in average dry mouth burden experienced per day over a period of 85 days after administration of an adrenergic receptor agonist and an antimuscarinic agent respectively.

Figure 5 is a flow chart illustrating an exemplary process of the present invention.

Figure 6 is a diagram of an exemplary computer system that maybe used to carry out the methods of the present invention.

DETAILED DESCRIPTION

Described herein is a computer-implemented method and system that is used to identify and analyze data reflecting adverse events or side effects (also generally referred to herein as burden) experienced by an individual (e.g., a patient or healthy volunteer taking part in a clinical trial, also referred to as a subject in the clinical trial setting) who is being treated with a medication for a condition (e.g., a condition, as used herein, refers to a disease or disorder). This methodology is noted as burden of therapy. In one exemplary embodiment, which is the setting of the examples described herein, the individual is a subject in a clinical trial. However, the invention is not so limited and can be used in other settings, i.e., those settings in which safety data is collected or when selecting a suitable therapy for an individual patient. The system and method may be utilized to compare the side effects experienced by two or more groups of comparable patients being treated by different type of medication for the same condition. The system and method may be utilized to compare the side effects experienced by an individual being treated with different types of medications for the same condition (e.g., a patient being treated for a condition using two different drugs, whereby the drugs can be administered concurrently, consecutively or sequentially), each having a different mechanism of action, or to compare the side effects experienced by two or more individuals being treated with the same medication, or to gain insight into the side effects experienced by an individual being treated with a single medication, these and other examples being described more fully herein.

Safety data from clinical studies are frequently reported as an absolute list or in summary fashion, which does not maximize the use of the detailed information included in a clinical study database. This provides only a limited viewpoint of the patient experience during the study, where the safety burden of treatment is only assessed as an overall summary over the study period without dissecting its true impact. Current methods do not allow the identification or differentiation of patients who experience more than one TEAE during a study, either at the same time or in a consecutive manner.

Thus, in the past, the burden was analyzed and presented by way of an overall indication of the incidence of a variety of side effects amongst individuals (e.g., subjects in a treatment group) being administered a particular medication. For example, prior art solutions identify the number of subjects (e.g., the percentage of the group) experiencing a particular side effect of a medication. Such prior art solutions do not distinguish between subjects that suffered an adverse event once and those subjects that suffered an adverse event throughout the entire course of treatment, or on multiple occasions during treatment.

Pursuant to the present invention, burden is analyzed on a per subject basis on each day of the treatment, which includes the length of time each adverse event is experienced, and, in preferred exemplary embodiments, includes a weighting associated with each adverse event (e.g., where the weighting is based on severity of the adverse event). Generally, the analysis could focus on adverse events associated with one patient per day, or groups of patients per day. Thus, in accordance with embodiments of the present invention, safety data is analyzed from the standpoint of the individual, and provides stakeholders (e.g., physicians, patients, payers and health authorities) with a clear and meaningful understanding of the true burden experienced by an individual or a group of individuals in order to evaluate the benefit of a therapeutic agent.

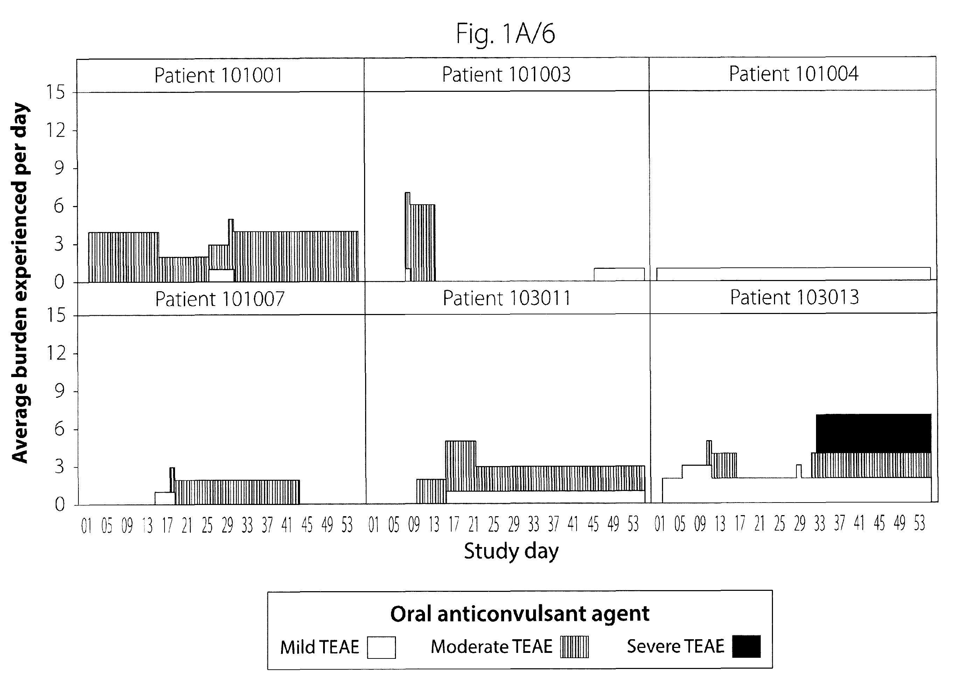

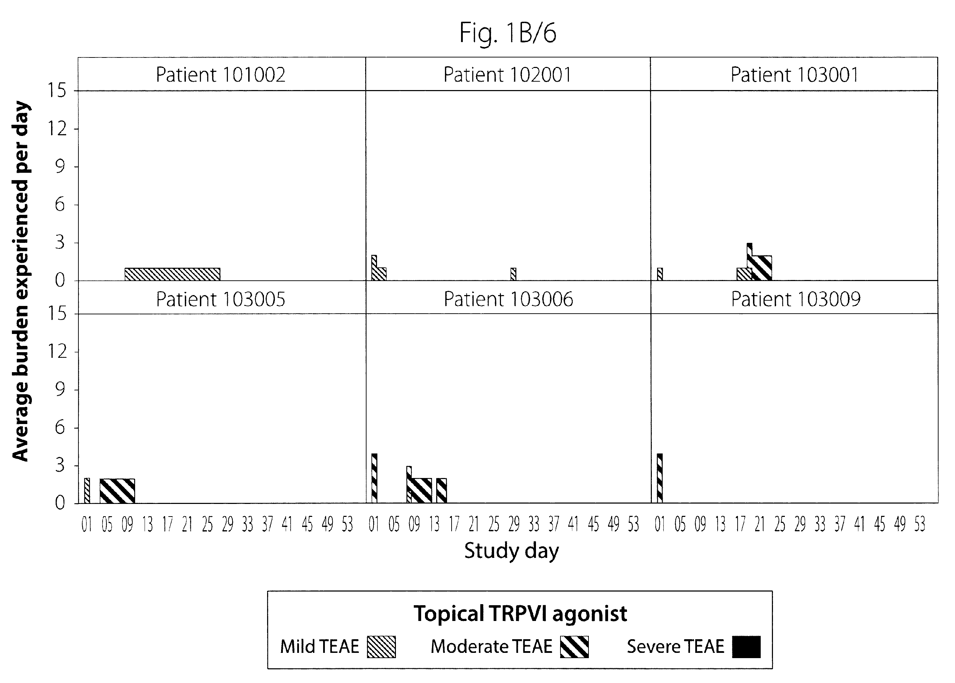

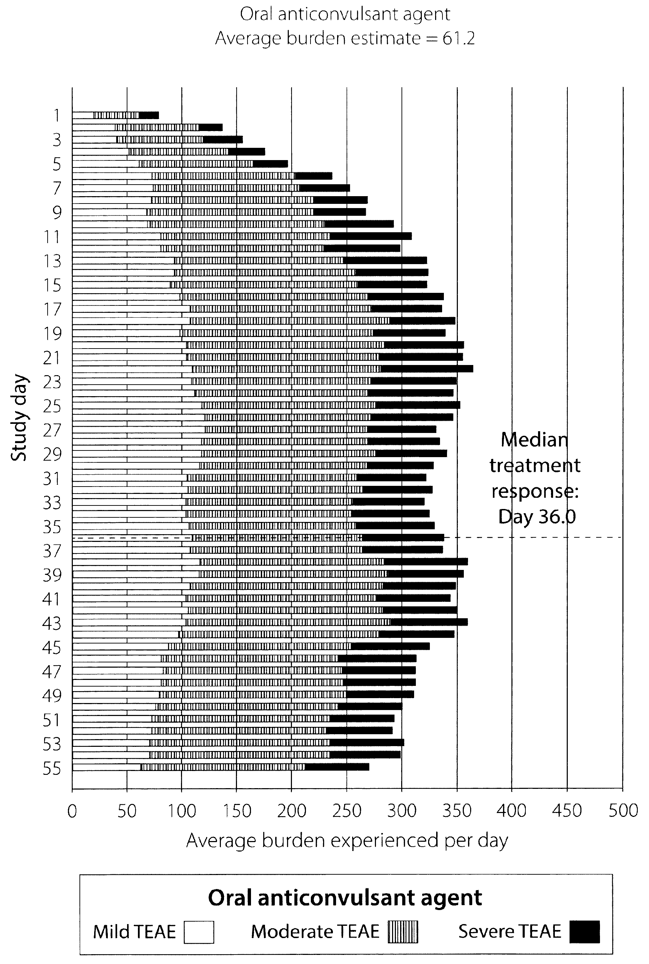

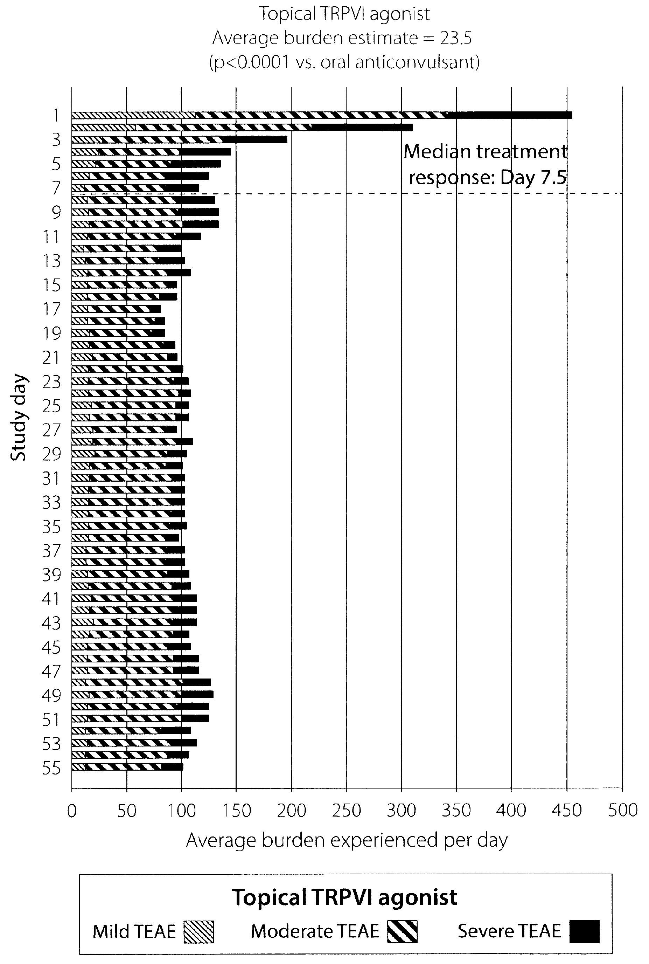

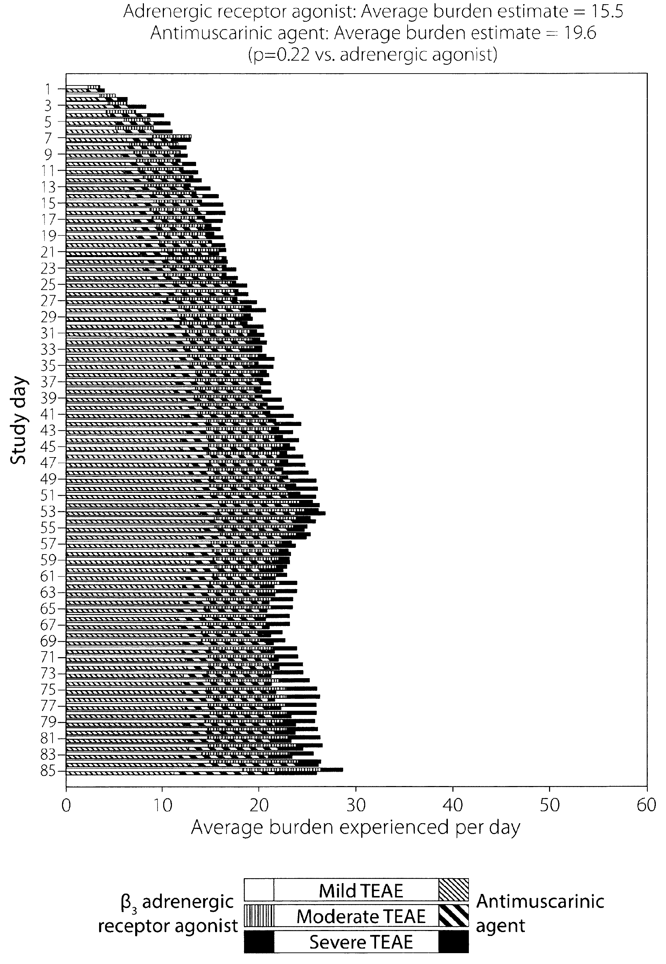

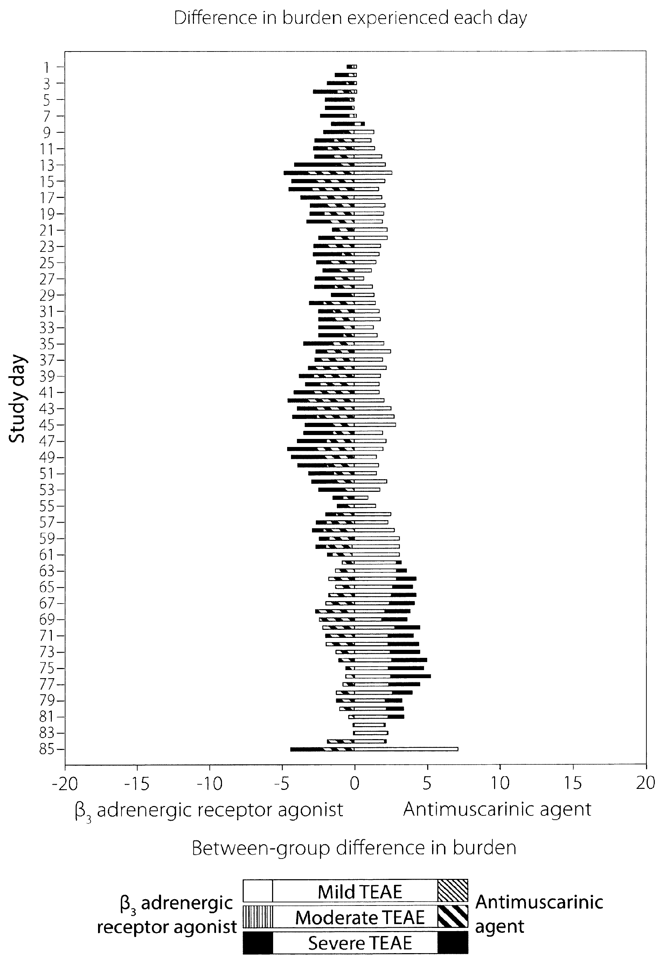

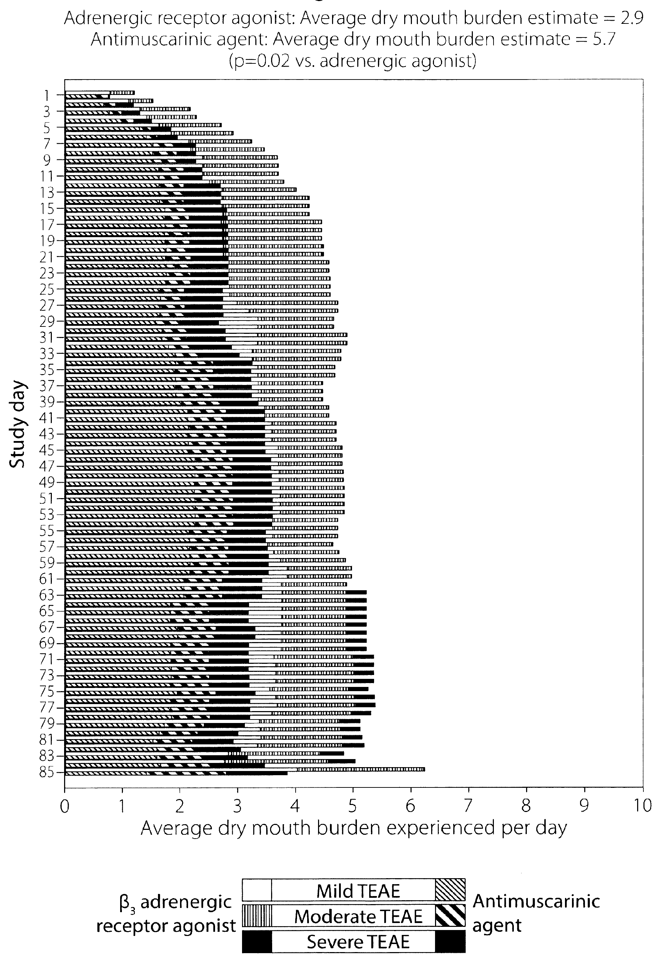

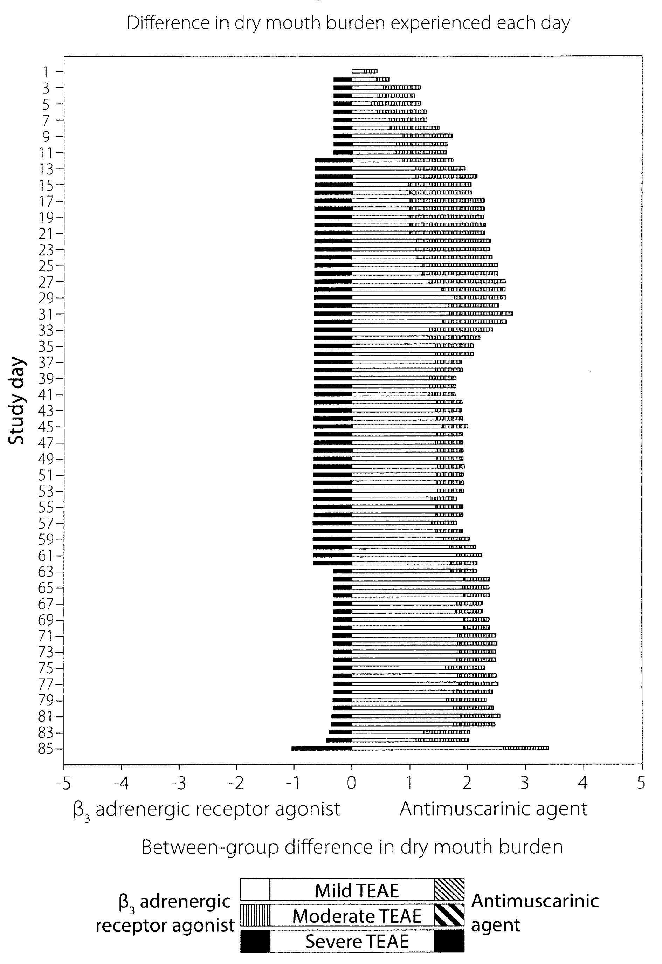

In accordance with embodiments of the system and method of the present invention, safety data is reported from a patient perspective, e.g., during a clinical study, referred to herein as the burden of therapy. Various examples of the application of the methodology to different clinical study databases show that it can be utilized to make novel safety observations/conclusions. For example, in a peripheral neuropathic pain study that investigated a topical vanilloid receptor subtype 1 agonist and an oral anticonvulsant agent, the topical group had a greater incidence of TEAEs than the oral group when evaluated using current methodology. Utilizing the burden of therapy methodology, it was shown that TEAEs with the topical agent were of short duration and occurred for 3 days after application, whereas TEAEs with the oral agent increased during dose titration and persisted to study end (see for example Figures 1A, 1B, 2A, 2B). In addition to analyzing safety, the burden of therapy methodology allows for an integrated analysis of clinical benefit and safety on each day during the clinical study. In an overactive bladder study that compared a $\beta_3$ adrenergic receptor agonist with an antimuscarinic agent, there was a minimal difference in overall TEAEs between groups, but the burden of therapy methodology revealed a higher burden of therapy related to dry mouth in the antimuscarinic group (see for example Figures 3A, 3B, 4A, 4B).

The burden of therapy methodology analyses the daily presence and severity of TEAEs experienced by patients during a clinical study. The methodology clearly provides a more accurate reflection of the burden of treatment from a patient perspective than current safety reporting. It is a highly sensitive and versatile tool and may also be applied in a personalized manner to the datasets of individual patients of interest, on TEAEs that affect specific body systems or during specific treatment periods. By integrating efficacy and safety into the analysis, it is possible to present the risk and benefit of a therapeutic agent throughout a clinical study. The burden of therapy methodology takes advantage of the wealth of information that is gathered during a clinical study and does not require additional data collection. The visual representation of burden of therapy aids in better informed treatment selection by clinicians and patients. The burden of therapy methodology has the potential to become the new standard for analyzing safety during clinical studies. The present invention is advantageous at least because it allows for:

- comparing various (one, two or more) different treatment regimens with respect to the burden of therapy/TEAEs associated with a given treatment regimen;
- comparing treatment vs. placebo and the burden of therapy/side effects associated with each;
- evaluating the effect of combination treatment of two different drugs for two or more different conditions to determine, e.g., whether there is an increase in side-effects;
- evaluating the TEAEs of treatments over time by following the patient during treatment, whereby the health care professional is checking the condition of the patient at certain intervals, to evaluate whether the dosing regimen should be adjusted, or after the treatment has been completed, to evaluate if there are any unexpected negative effects as a consequence of the treatment.
- assessing the risks of a treatment during a time period of interest, including when or whether any deaths occur;
- assessing efficacy and TEAEs with a new chemical compound in an early phase of a clinical trial;
- allowing a medical doctor to discuss treatment profiles with the patient including which treatment would be acceptable to the patient;
- adjusting the efficacy dose of a therapeutic for each single individual while also considering the adverse event data for that individual;
- allowing health care professionals to continue to follow patients after treatment to gather additional data of the patient (particularly follow up on TEAEs);
- adding information regarding a potential treatment failure;
- capturing the natural history of any disease beyond treatment failure endpoints (events); and
- assessing disease progression, especially when "treatment failure" outcome measures are used, in order to provide valuable insight into the TEAEs experienced by patients, as well as the burden related to treatments such as chemotherapy that may continue after patients had reached such endpoints; and combinations of at least certain of the foregoing.

The foregoing examples are exemplary only and do not constitute limits on the present invention.

The methodology utilizes patient-level safety data to derive a quantitative estimate for the burden of adverse events that all patients experience on each day of a clinical study. The burden estimate considers adverse event severity, any serious event and any event leading to drug discontinuation, in the preferred embodiment. A chart may be generated to show the total burden experienced by patients on each day throughout the study. The area under curve represents the total burden experienced by patients. Exemplary implementations of the present invention are described herein involving two validated and published clinical studies in peripheral neuropathic pain and overactive bladder. It has been found that the burden of therapy methodology permitted novel safety observations from each of the clinical studies. The presence of TEAEs during the peripheral neuropathic pain study varied between arms during the study. TEAEs occurred immediately after treatment and rapidly reduced in the topical group, while in the oral group, TEAEs gradually increased in presence from study start to end.

The integration of efficacy and safety in the burden of therapy graph demonstrates the versatility of the methodology.

The methodology of the present invention begins by obtaining the data reflecting any side effects experienced by a subject. More particularly, for a single subject, it is determined whether the subject experienced one or more TEAEs on each day of treatment (e.g., of the study). A questionnaire may be used to gather such data, an example of which is attached hereto as Appendix A, the entire contents of which are hereby incorporated by reference. This questionnaire also describes the logic that may be used to process the data collected using the questionnaire. Other manners of collecting the data may be used in accordance with the present invention. The data, once obtained from the patient, may be stored in a database file. Ultimately, in the preferred embodiment a SAS® data set must be created using the collected data, either by creating a SAS® data file (i.e., a data set that holds actual data), or a SAS® view (i.e., a data set that references data that is stored elsewhere, e.g., in a database). More particularly, in an exemplary embodiment, the individual subject adverse event data is typed into an electronic Case Record Form (eCRF) and, from there, directly handled and stored in a database. Different database systems can be used for collecting and storing adverse events information. In whatever database the adverse events data is stored, it is transferred to SAS® datasets. SAS® can load the data from many database formats such as ORACLE, Excel, csv, Microsoft ACCESS, DBF, XML and PARADOX. A SAS® data set is a SAS® file stored in a SAS® library that SAS® creates and processes. A SAS® data set contains data values that are organized as a table of observations (rows) and variables (columns) that can be processed by SAS® software. Variables, as used herein, refers to containers that are created within a program to store and use character and numeric values. Variables have attributes, such as name and type, for identifying them and that define how they can be used. A variable name would, for example, be "AGE" which is numerical. The variable would contain the age value of patients in a study. A SAS® data set also contains descriptor information such as the data types and lengths of the variables, as well as which engine was used to create the data.

Adverse events are reported by the individual to the medical doctor (i.e., what type of adverse event was experienced by the individual, if any, on each given day). The adverse events so reported are categorized by the doctor, in terms of severity, as mild, moderate and severe. In an exemplary embodiment, the doctor may use the following criteria for defining the severity of an adverse event including abnormal clinical laboratory values: (1) mild - no disruption of normal daily activities; (2) moderate - affect normal daily activities; and (3) severe - inability to perform daily activities. Each severity grading may be associated with a severity indicator. In the exemplary embodiment, a severity indicator is an indicator of severity of an adverse event, for example, expressed as an ordinal scale, e.g., grades 1 - 5. The severity indicator may be used in connection with weighting the adverse events. For example, in one embodiment, the number of mild adverse events is multiplied by 1, the number of moderate adverse events is multiplied by 2, and the number of severe adverse events is multiplied by 3. This weighting schema is exemplary; other methods for weighting the adverse events according to severity are within the scope of the present invention. For example, in connection with some studies (such as those described in the examples described herein), a five grade scale may be used for indicating severity, with grade 4 being life-threatening; urgent intervention indicated, and grade 5 is death related to an adverse event.

A seriousness indicator, associated with the adverse events, may also be used, indicating the seriousness of the adverse event.

The results of the data processed in accordance with the foregoing can be rendered in a variety of graphical displays. In one embodiment, for a given medication, a stacked bar is created for each day that shows the number of mild adverse events x 1 in one color; the number of moderate adverse events x 2 in a second color; and the number of severe adverse events x 3 in a third color. A particular color scheme may be chosen for the medication (e.g., an orange color scheme). This same process can be repeated for other medications being administered to the individual, if more than one, which can then be used for comparisons across multiple medications. Such other medication(s) can be reflected in another color scheme (e.g., a blue color scheme) and, in this case, the stacked bars for each day for each medication can be plotted on the same graph. This allows for a visual comparison of the adverse events associated with the two medications. Instead of, or in addition to, different colors, different shades of colors can be used to reflect the mild, moderate and severe adverse events or, as shown in the figures herein, different black and white fillings reflecting the mild, moderate and severe adverse events.

In other variations of embodiments of the invention, stacked bars are not used and, instead, the number of subjects with a certain adverse event per day is plotted in a normal graph versus time in days. In still other variations, the number of subjects with a treatment-related adverse event (i.e., determined by the doctor) per day is plotted.

In other embodiments, a graph may be rendered representing the sum of weighted adverse events for all patients for each day that the subjects are in the clinical trial divided by the number of patients participating in the trial. In another embodiment, a graph may be generated that presents the number of patients with an adverse event colored by the worst severity on that day, divided by the number of patients at risk. In a further embodiment, a graph may be generated that presents the number of patients with an adverse event colored by adverse events of interest. In case multiple adverse events of interest are reported, then the coloring will be equally spread over the adverse events at risk for that patient, divided by the number of patients at risk.

In still other embodiments of the invention, the sum of the duration that a subject has an adverse event, e.g., in days, is shown. In some embodiments of the invention, a severity weighting of 1 is used for all adverse events although, from a medical perspective, weighting is an important aspect of the analysis.

Once the graph(s) reflecting the adverse event data are generated, additional data can be added to the graphs to allow for more detailed statistical analysis. For example, a line indicating the median response time (i.e., the median time within which subjects in the study respond to treatment with the medication) can be plotted.

The systems and methods of the present invention are now described with reference to specific examples of clinical trials and the adverse event data generated in connection therewith, and stored in a database. The first involves a peripheral neuropathic pain database (ClinicalTrials.gov Identifier: NCT01713426; Haanpää et al., Eur J Pain 2015. Epub. DOI: 10.1002/ejp.731) with a study of a topical agent and an oral agent; the second an overactive bladder database (NCT01638000; Batista, J.E., Kölbl, H., Herschorn, S., et al. The efficacy and safety of mirabegron compared with solifenacin in overactive bladder patients dissatisfied with previous antimuscarinic treatment due to lack of efficacy: results of a non-inferiority, randomized, phase IIIb trial. Ther Adv Urol August (7), p. 167-179, 2015; [Epub first published 30 June 2015]. DOI: 10.1177/1756287215589250) with a $\beta_3$ adrenergic receptor agonist and an anti-muscarinic agent. General names for each drug treatment are used herein.

First described is the data analysis involved with implementation of the invention. More particularly, the application of the methodology involves the use of a clinical study database with individual data entries for TEAEs per patient per day. The presence of TEAEs may be plotted against the study day, such that if a patient reported a TEAE from Days 3 to 5, this TEAE was considered present on Days 3, 4 and 5. Also, if an end date was not present in the database, or a TEAE was ongoing, the last study day of the patient was taken as the end day of the TEAE. The graph that is produced from the data displays the number of patients with a TEAE on each study day. The addition of coloring that corresponds with TEAE severity allows easy discrimination of TEAEs of differing severity in the graph.

In order to visually represent the safety/TEAE burden experienced by a patient, varied weighting may be applied to TEAE severity in each arm in a consistent manner. The graph may, therefore, display the number of weighted TEAEs per day. For purposes of example, in the graphs described herein, TEAEs that were mild, moderate, severe, life threatening/disabling and related to death were weighted to represent 1x, 2x, 3x, 4x and 5x TEAEs per event per day, respectively. Further, the investigator may indicate the seriousness of the TEAE.

For example, a serious TEAE may be any untoward medical occurrence that, at any dose (even a 0 dose (= placebo)), results in death; is life threatening (an event in which the subject is at risk of death at the time of the event, although it does not refer to an event which hypothetically might have caused death if it was more severe); results in persistent or significant disability/incapacity; results in congenital anomaly or birth defect; requires in-subject hospitalization or leads to prolongation of hospitalization; or other medically important events.

An area under the curve (AUC) analysis can be performed in conjunction with the burden of therapy methodology to quantitatively compare potential differences in mean events per day between study arms. This can be performed in conjunction with weighting of events if required. An AUC analysis may be useful in studies that have a similar presence of events in each arm, as an estimate of the mean weighted events per day for each arm can be compared using statistical tools.

In addition to the presentation of TEAEs, the burden of therapy graph can also be integrated with an efficacy analysis. By displaying both the efficacy outcome over time and weighted TEAEs over time in one graph, it is possible to directly compare the effectiveness of treatments and their burden on the patient in a single analysis. Any efficacy endpoint that has been recorded by time may be used for this type of analysis. Such integrated efficacy and safety graphs visually describe the cost and benefit of treatment from the patient point of view. Exemplary integrated graphs with TEAEs and efficacy are described herein (Figures 2A and 2B).

The burden of therapy graph may be produced using the SAS@ program, as mentioned previously. The preferred exemplary format for the graph is a mirrored display bar chart with study day on the y-axis and number of TEAEs on the x-axis, for example as shown in Figure 2A and Figure 2B. This design allows convenient comparison of the adverse event profile per day of each treatment along the horizontal x-axis. Alternatively, study day can also be on the x-axis and adverse event profiles can be compared along the vertical y-axis, for example as shown in Figures 1A and 1B.

An alternative exemplary format for the graph is to overlay two treatments on top of each other as shown in Figures 3A and 4A. This can be done to compare two treatments that have a similar safety profile and in conjunction with a comparison of the mean events per day. In addition, the difference in TEAEs per day and per severity (mild, moderate, severe) between treatments can also be plotted to help compare similar treatments (see Figures 3B and 4B). One disadvantage of this manner of displaying the data may be that the graph looks very busy and it is more difficult to see the TEAE profile from one of the two treatment arms. Thus, this display may be used if the two profiles are very similar and differentiation between the two profiles can only be seen if one is displayed over the other.

The addition of an efficacy outcome into the graph may be in a line graph format and in a different color to that used for TEAEs.

The mathematical rules applied to derive the burden of therapy graph are described below, where the burden on each day for an individual subject is $\text{Burden}_{ij}$:

$$burden_{ij} = \sum_{v=1}^{k} weight_{vij}.AE_{vij}$$

- $\text{AE}_{vij}=1$ if an AE is present for subject i on day j,
- $\text{AE}_{vij}=0$ if an AE is not present for subject i on day j

and

$$weight_{vij} = \prod_{u=1}^{h} weight\_element_{uvij}.$$

$\text{Burden}_{ij}$ is the burden of subject i on day j based on the k adverse events that this subject experienced. $\text{Weight}_{vij}$ is the weight of the *vth* AE that subject i experienced on day j. $\text{Weight}_{vij}$ is the product of the different weight elements ($\text{weight\_element}_{uvij}$), such as severity, drug discontinuation and seriousness. The h in the equation is the number of weight_element factors taken into account. Thus, $\text{weight\_element}_{uvij}$ is the u*th* weight element of weight v for subject i on day j.

Each bar in the burden of therapy graph represents the total burden of all subjects in the study divided by the subjects at risk, and can be derived by the sum of the burden of each individual subject divided by the subjects in the study on day j ($n_j$): $\text{bar}_j = \frac{\sum_{i=1}^{n_j} burden_{ij}}{n_j}$.

The graph is generated with each bar with length $\text{bar}_j$, with the order as day number.

The AUC for each individual subject can also be calculated. The AUC represents the total burden that an individual subject experienced during the trial. The AUC is the sum of the burden, for all burdens of an individual subject. Thus, the AUC of subject i is $\text{burden}_i = \sum_{j=1}^{t_i} burden_{ij}$, where $t_i$ is the total number of study days of subject i.

The AUC can be used in an analysis of covariance (ANCOVA) as a response variable and the impact of different covariates and factors on the AUC can be investigated. The obvious factor is treatment, but one can include medication history, age, gender or BMI as response variables in the ANCOVA analysis.

The results of the exemplary studies are now described.

In the peripheral neuropathic pain study, the total number of patients with TEAEs was 210 (74.5%) in the topical agent arm (Topical TRPVI agonist) and 177 (63.9%) in the oral agent arm (Oral anticonvulsant agent) (Haanpää et al., Eur J Pain 2015. Epub. DOI: 10.1002/ejp.731).

Figure 1A shows the graphs of 6 subjects individually treated with the oral agent and Figure 1B shows the graphs of 6 subjects treated with the topical agent. All 12 subjects in Figures 1A and 1B have some TEAE. A burden of therapy analysis can be performed on an individual patient level and such an analysis during this study showed that the time and severity that subjects experience these TEAEs in the study is different in the oral agent arm than in the topical agent arm. A greater number of TEAEs were experienced for a greater number of days with the oral agent arm versus the topical agent arm (Figure 1A and Figure 1B).

The burden of therapy graph shows that there was a noticeable difference in the presence of events in the two groups as the study progressed. In the topical group, there was an initial peak followed by a rapid decline in weighted TEAEs per day (Figure 2B), as they were primarily application site reactions of short duration. The overall burden estimate was 61.2 for oral group and 23.5 for the topical group. The burden of therapy graph highlighted the contrasting severity of TEAEs in the oral versus topical groups. There were more weighted moderate and severe TEAEs in the oral compared with topical group from Days 5-55. TEAE weighting in both arms allowed this to be more easily visualized as the representation of all moderate or severe TEAEs increases in both arms (Figures 2A and 2B).

The integration of safety and efficacy into the burden of therapy graph allowed the safety burden to be directly compared with the efficacy benefit of treatment during the clinical study. In this study, median time to treatment response was compared and this was defined as the first of three consecutive days in which the patient reported a $\geq$30% reduction in average daily pain score (Haanpää et al., 2015). With the topical TRPV1 agonist, patients rapidly achieved this median treatment response (Day 7.5), and this overlapped with the period that transient application site reactions were observed and then stabilized to a low background level (reported by <5%). With the oral anticonvulsant agent, the median treatment response was more gradual (Day 36.0) and this period coincided with dose-titration and a gradual increase in reporting of drug-related TEAEs to a moderate-to-high background level (reported by 20-39%) (Figures 2A and 2B).

The burden of therapy methodology was also useful when applied to an overactive bladder study that compared a $\beta_3$ adrenergic receptor agonist with an antimuscarinic agent. In this study, the total number of patients with TEAEs was 274 (29.3%) in the $\beta_3$ adrenergic receptor agonist group and 282 (30.2%) in the anti-muscarinic agent group (Batista et al., 2015. Epub. DOI: 10.1177/1756287215589250). Using the burden of therapy methodology, the burden per day during the study was similar in both groups and differences between the groups were not immediately noticeable in the chart (Figures 3A and 4A). The overall burden estimate (prior antimuscarinic use as acovariate) was 19.6 for the antimuscarinic agent and 15.1 for the $\beta_3$ adrenergic receptor agonist (p=0.22 for between-group comparison). In order to help visualize the difference between treatment groups, the percentage of patients with a TEAE per day in each group was overlaid in a single plot and the difference was also plotted (Figures 3B and 4B). Using this variation of the burden of therapy methodology, it was possible to identify that during the study there was a small difference in the proportion of patients with TEAEs in the antimuscarinic agent group, which gradually increased from 0.1% at Day 10 to 5% by study end at Day 85.

Anti-muscarinic agents are known for specific side effects like dry mouth, constipation and headache. The burden of therapy methodology was used to focus on this specific side effect only. In Figures 3B and 4B, one can see the profiles of dry mouth in this study.

To analyze the profiles with an ANCOVA, the AUC for dry mouth of each subject was derived. The AUC was then used as the dependent variable in the model. Interestingly, in this study, data was collected for each subject if they had some negative experience with the use of anti-muscarinic treatment in their past, called prior anti-muscarinics dissatisfaction. It is likely that a negative past experience with anti-muscarinics could influence the recording of a specific adverse event like dry-mouth. In the statistical (ANCOVA) model, prior anti-muscarinics dissatisfaction was added next to treatment as explanatory variable. The result of the statistical analysis showed statistical significance levels for treatment differences (p=0.02) meaning that more subjects had a dry mouth using anti-muscarinics. Prior anti-muscarinic dissatisfaction had also a low p-value (p=0.007), meaning that subjects with a negative experience using anti muscarinics also reported more dry mouths. In this study, dry mouth was reported by 29 patients (3.1 %) in the $\beta_3$ adrenergic agonist group and 54 patients (5.8%) in the antimuscarinic group (Batista et al., 2015). An analysis of patients who reported dry mouth was performed to investigate whether there was a difference between arms and approximately 1.5-2.0% of patients reported dry mouth in the antimuscarinic group from Days 17-85 (Figures 3B and 4B). The burden estimate (prior antimuscarinic use as a covariate) from dry mouth was 5.7 with the antimuscarinic agent and 2.9 for the adrenergic receptor agonist (p=0.02 for between-group comparison). This demonstrated that there was a statistically significant difference between treatment groups and that it was more likely that patients reported dry mouth in the antimuscarinic group than in the $\beta_3$ adrenergic receptor agonist group.

The example above shows that deriving an AUC using the burden of therapy methodology allows for formal statistical analysis, taking the TEAE profile into account for each individual subject. This can be done for all reported TEAEs, as well as a subset of interest, such as dry mouth in the example above.

In view of the foregoing, it can be seen that the burden of therapy methodology is a novel method that can be used to report daily safety data for the entire duration of a clinical study, thereby more accurately reflecting the burden of therapy related adverse effects experienced by patients. As described herein, the methodology was applied to two different validated clinical study databases and several new safety conclusions were made and quantitatively analyzed in greater detail.

For example, with the peripheral neuropathic pain study, the methodology allowed the observation that the presence of TEAEs was substantially different between the topical and oral agent groups, which was not possible with the traditional safety reporting of overall incidence for the study (Haanpää et al 2015).

A simple scale of TEAE weighting (1 for mild, 2 for moderate and 3 for severe) was applied in the Burden of Therapy methodology in order to more accurately reflect the clinical impact and burden of TEAEs on a patient during a study. In clinical practice, TEAEs of different severities can have a very different level of impact on a patient. By not applying weighting criteria, a weighting of one is assumed for all TEAEs and it is also assumed that all TEAEs have an equal impact or burden on the patient. In theory, additional weighting could be used in order to reflect the seriousness in addition to the severity of TEAEs. Also, a weighting method could in the future be standardized for a particular development program, therapeutic area, or patient population, or particular diseases. Despite this, the relative advantages and disadvantages of the weighting scale of TEAEs used in this paper may be a point of consideration when this methodology is utilized and other means or criteria of weighting TEAEs may be considered.

The combined safety and efficacy example of the burden of therapy methodology highlights the utility and adaptability of the methodology. By using the combined burden of therapy graph, it is possible to track the burden and benefit of treatment at any particular day throughout a clinical study.

The burden of therapy can also be utilized in a personalized manner as the datasets for individual patients of interest, specific patient populations, or TEAEs that affect specific body systems, or within specific timelines of a study, can all be analyzed individually using the methodology. Therefore, the burden of therapy methodology may help healthcare to achieve personalized treatment that may be well tolerated in specific patient subgroups.

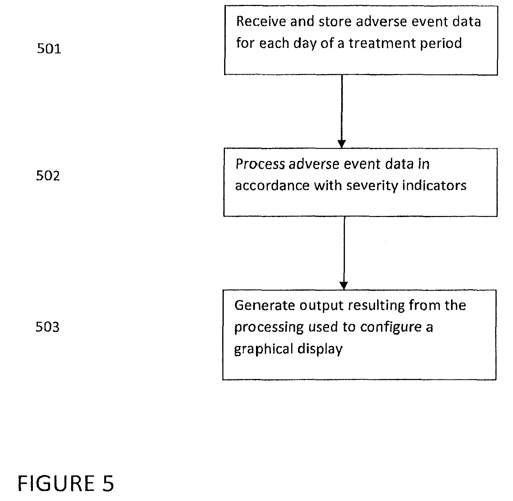

Figure 5 is a flow chart illustrating an exemplary process of the present invention. In step 501, adverse event data for each day of a treatment period is received and stored. In step 502, adverse event data is processed in accordance with severity indicators. In step 503, an output resulting from the processing is generated and used to configure a graphical display.

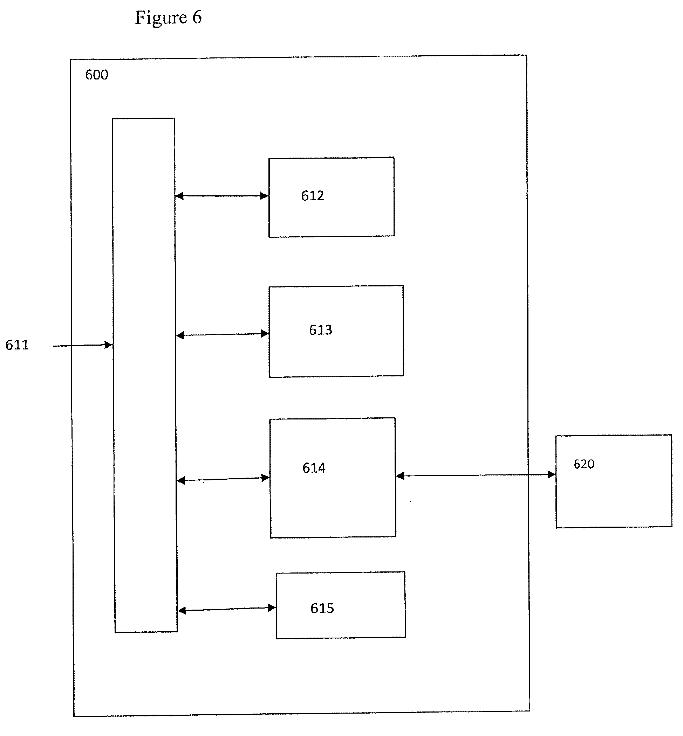

Computer system 600, as shown in Figure 6, may be used to implement the methods of the present invention, which methods may be implemented as programmable code for execution by computer system 600. More particularly, computer system 600 comprises hardware, as described more fully herein, that is used in connection with executing software/computer programming code (i.e., computer readable instructions) to carry out the steps of the methods described herein.

Computer system 600 includes one or more processors 612. Processor 612 may be any type of processor, including but not limited to a special purpose or a general-purpose digital signal processor. Processor 612 may be connected to a communication infrastructure 611 (e.g. a data bus or computer network) either via a wired connection or a wireless connection. Communication infrastructure 611 carries signals and may be implemented using wire or cable, fiber optics, a phone line, a wireless link, a cellular phone link, a radio frequency link, or any other suitable communication channel, including a combination of the foregoing exemplary channels.

Computer system 600 includes one or more memories 613. Memory 613 may include at least one of: random access memory (RAM), a hard disk drive and a removable storage drive, such as a floppy disk drive, a magnetic tape drive, or an optical disk drive. The removable storage drive reads from and/or writes to a removable storage unit. The removable storage unit can be a floppy disk, a magnetic tape, an optical disk, which is read by and written to a removable storage drive.

In alternative implementations, memory 613 may include other similar means for allowing computer programs or other instructions to be loaded into computer system 600. Such means may include, for example, a removable storage unit and an interface. Examples of such means may include a removable memory chip (such as an EPROM, or PROM, or flash memory) and associated socket, and other removable storage units and interfaces which allow software and data to be transferred from removable storage unit to computer system 600. Alternatively, the program may be executed and/or the data accessed from the removable storage unit, using the processor 612 of the computer system 600.

Computer system 600 includes one or more user interfaces 614. User interface 614 may be a program that controls a display (not shown) of computer system 600, on which the output of the processes described herein (e.g., the graphs generated by the inventive processes) can be displayed. User interface 614 may include one or more peripheral user interface components, such as a keyboard or a mouse. The end user may use the peripheral user interface components to interact with computer system 600. User interface 614 may receive user inputs, such as mouse inputs or keyboard inputs from the mouse or keyboard user interface components.

In some embodiments, user interface 614 displays data on the display of computer system 600 using a web browser. A web browser may be an application with the ability to render HTML pages, Cascading Style Sheets (CSS) and JavaScript content on the display of user interface 614. In some embodiments, user interface 614 displays data, such as web pages, on the display of client device 620 using another software application. One of ordinary skill in the art will appreciate that user interface 614 is not limited to displaying data using a web browser or another software application, and that embodiments of the present invention may contemplate using other display devices or software suitable for the purposes of the displaying the data.

Computer system 600 may also include a communication interface 615. Communication interface 615 allows software and data to be transferred between computer system 600 and an external device 620. Examples of communication interface 615 may include a modem, a network interface (such as an Ethernet card), and a communication port, by way of example. Software and data transferred via communication interface 615 are in the form of signals, which may be electronic, electromagnetic, optical, or other signals capable of being received by communication interface 615. These signals are provided to communication interface 615 via a communication infrastructure 611.

Thus, in exemplary embodiments, there is included one or more computers having one or more processors and memory (e.g., one or more nonvolatile storage devices). In some embodiments, memory or computer readable storage medium of memory stores programs, modules and data structures, or a subset thereof for a processor to control and run the various systems and methods disclosed herein. In one embodiment, a non-transitory computer readable storage medium having stored thereon computer-executable instructions which, when executed by a processor, perform one or more of the methods disclosed herein.

With reference now to embodiments of the particular invention, computer system 600 may be specially programmed using statistical analysis software to implement the methodologies described herein. The statistical analysis software may be programmed to implement the logic described in the questionnaire attached hereto as Appendix A. Data files, containing the data responsive to the questionnaire for each patient, may be read by computer system 600 and processed to produce an output. Such output may be data capable of being displayed in the form of graphs, as described in more detail herein, via user interface 614.

A computer is required to perform the methodologies as described. The methodologies could not be performed manually because of the number of data records in a dataset containing the adverse event information and the number of derivation step each record require. A simple study includes a few hundred data lines, whereas a larger trial could easily contain 10s of thousands records. Each of these data records must be processed using the derivation logic (patient-level and treatment-level derivation described previously). For example, if a three month study contains 10,000 records, the number of derivations easily exceeds 1,000,000. Processing over 1,000,000 records will be ineffectively time-consuming, requiring about 18 years when working 24 hours a day. Also, the computer system is used to generate a graphical display of the processed data. Until the present invention, adverse event data was not processed using a computer in this way, nor was it presented using the graphical displays described herein.

It will be appreciated by those skilled in the art that changes could be made to the exemplary embodiments shown and described above without departing from the broad inventive concept thereof. It is understood, therefore, that this invention is not limited to the exemplary embodiments shown and described, but it is intended to cover modifications within the spirit and scope of the present invention as defined by the claims. For example, specific features of the exemplary embodiments may or may not be part of the claimed invention and features of the disclosed embodiments may be combined.

It is to be understood that at least some of the figures and descriptions of the invention have been simplified to focus on elements that are relevant for a clear understanding of the invention, while eliminating, for purposes of clarity, other elements that those of ordinary skill in the art will appreciate may also comprise a portion of the invention. However, because such elements are well known in the art, and because they do not necessarily facilitate a better understanding of the invention, a description of such elements is not provided herein.

Further, to the extent that the method does not rely on the particular order of steps set forth herein, the particular order of the steps should not be construed as limitation on the claims. The claims directed to the method of the present invention should not be limited to the performance of their steps in the order written, and one skilled in the art can readily appreciate that the steps may be varied and still remain within the spirit and scope of the present invention.

APPENDIX A

Questionnaire

Patient level questions:

Questions about the adverse events

- Did the patient report any TEAE? (Yes/No)
- What was the TEAE reported?
- When did the TEAE started (study day)?
- When was the TEAE resolved (study day)?
- What was the worst severity (mild, moderate or severe)?
- Was hospitalization required (Yes/No)?
- Was this a serious TEAE (Yes/No)?
- Did the TEAE result in death (Yes/No)?
- Was there a treatment interference because of this TEAE (Treatment is stopped/Treatment is interfered/None)?

Derivation logic

- When was the investigational drug or it's control used for the first time (date)?
- Define the first treatment day as day 1: on which days did the TEAE occur?
- What is the weight for this TEAE on day x? (for example, if severity is the weight factor than the weight is mild=1, moderate=2 and severe=3)
- Other weight variables:
- Death is always a weight of 10, whatever the other parameters indicate.
- Do you have an TEAE: multiply by 0 or 1 (1 is yes)
- Hospitalisation: multiply by 1 or 2 (2 is yes)
- Serious: multiply by 1 or 4 (4 is yes)
- Treatment interference: 1, 2, 3 (2 is temporary stop of treatment and 3 is stop treatment)
- For each day what is the sum of the weight factors for patient i.
- On which day did the patient respond to treatment

Treatment group level

D erivation logic

- What is the sum of the weighted TEAE's for day x?
- For each study day draw a bar where the height is the sum of weighted TEAE's of that day.
- How many patients are at risk on day x (number of patients that are in the study on treatment j on day x)?
- How many of the patients did respond to treatment from day 1 till day x?
- For each day draw the proportion of patients that did respond since day 1, given the number of patients at risk.
- What was the median for the time till response.
- Multiply the result of each question and divide that by 10. So the worst case is death, second worst is someone that has a severe, serious TEAE that caused a stop of treatment and hospitalization. That is 3x4x3x2=72/10=7.2 so a weight of 7.2 for that TEAE for that day.

**Claims**

1. A system comprising:

at least one non-transitory memory configured to store adverse event data, said adverse event data describing whether an individual experienced an adverse event as a result of being administered a therapeutic agent on each day during a time period; and

at least one processor, operatively connected to the at least one non-transitory memory, configured to process the adverse event data and generate an output.

2. The system of claim 1 wherein the adverse event data comprises at least one severity indicator associated with the adverse event experienced on each of the days in the time period.

3. The system of claim 2 wherein the severity indicator comprises one of mild severity, moderate severity, and severe severity.

4. The system of claim 3 wherein the severity indicator is associated with a weight.

5. The system of claim 3 wherein the severity indicator for mild severity is 1, the severity indicator for moderate severity is 2, and the severity indicator for severe severity is 3.

6. The system of claim 2 wherein the adverse event is death and the severity indicator is associated with a weight of 5.

7. The system of claim 2 wherein the severity indicator comprises a hospitalization indicator, describing whether the adverse event was associated with hospitalization.

8. The system of claim 2 wherein a seriousness indicator is processed, describing the level of seriousness associated with the adverse event.

9. The system of claim 8 wherein the severity indicator comprises a treatment interference indicator, describing whether treatment continued without interruption, was stopped temporarily, or was stopped permanently.

10. The system of claim 2 wherein the processing the adverse event data comprises associating a weight with each of the one or more severity indicators and summing the weights for each day.

11. The system of claim 1 wherein the non-transitory memory is further configured to store data describing a day, within the time period, on which the individual responded to the therapeutic agent.

12. The system of claim 1 wherein the adverse event data is collected using a questionnaire.

13. The system of claim 1 wherein the output is used to generate a graphical display.

14. The system of claim 1 wherein a plurality of individuals are being administered the therapeutic agent on each of the days during the time period, and wherein the processor is further configured to: determine how many individuals are being administered the therapeutic agent on a given day during the time period; determine how many of the individuals being administered the therapeutic agent on the given day responded to the treatment by the given day; determine a proportion of individuals that responded to the treatment by the given day out of a total number of the plurality of individuals; and determine a median time for response to the treatment for the plurality of individuals.

15. Use of a computer processor for:

storing adverse event data in a database, said adverse event data describing whether an individual experienced an adverse event as a result of being administered a therapeutic agent on each day during a time period; and processing the adverse event data and generate an output.

16. The use of claim 15 wherein the adverse event data comprises at least one severity indicator associated with the adverse event experienced on each of the days in the time period.

17. The use of claim 16 wherein the severity indicator comprises one of mild severity, moderate severity, and severe severity.

18. The use of claim 17 wherein the severity indicator is associated with a weight.

19. The use of claim 17 wherein the severity indicator for mild severity is 1, the severity indicator for moderate severity is 2, and the severity indicator for severe severity is 3.

20. The use of claim 16 wherein the adverse event is death and the severity indicator is associated with a weight of 5.

21. The use of claim 16 wherein a seriousness indicator is processed, describing the level of seriousness associated with the adverse event..

22. The use of claim 21 wherein the seriousness indicator comprises a hospitalization indicator, describing whether the adverse event was associated with hospitalization.

**23.** The use of claim 16 wherein the severity indicator comprises a treatment interference indicator, describing whether treatment continued without interruption, was stopped temporarily, or was stopped permanently.

**24.** The use of claim 16 wherein the processing the adverse event data comprises associating a weight with each of the one or more severity indicators and summing the weights for each day.

**25.** The use of claim 15 wherein the storing further comprises storing data describing a day, within the time period, on which the individual responded to the therapeutic agent.

**26.** The use of claim 15 wherein the adverse event data is collected using a questionnaire.

**27.** The use of claim 15 wherein the output is used to generate a graphical display.

**28.** The use of claim 15 wherein a plurality of individuals are being administered the therapeutic agent on each of the days during the time period, for:

> Determining how many individuals are being administered the therapeutic agent on a given day during the time period;
> Determining how many of the individuals being administered the therapeutic agent on the given day responded to the treatment by the given day;
> determining a proportion of individuals that responded to the treatment by the given day out of a total number of the plurality of individuals; and
> determining a median time for response to the treatment for the plurality of individuals.

**29.** A non-transitory computer readable storage medium storing instructions for a computer processor used according to any one of claims 15 - 28.

# Average burden experienced per day

Fig. 1A/6

Fig. 1B/6

Oral anticonvulsant agent
Average burden estimate = 61.2

**Oral anticonvulsant agent**

Mild TEAE ☐   Moderate TEAE ▥   Severe TEAE ■

Topical TRPVI agonist
Average burden estimate = 23.5
(p<0.0001 vs. oral anticonvulsant)

Average burden experienced per day

**Topical TRPVI agonist**

Mild TEAE   Moderate TEAE   Severe TEAE

Adrenergic receptor agonist: Average burden estimate = 15.5
Antimuscarinic agent: Average burden estimate = 19.6
(p=0.22 vs. adrenergic agonist)

Difference in burden experienced each day

Between-group difference in burden

EP 3 125 138 A1

Adrenergic receptor agonist: Average dry mouth burden estimate = 2.9
Antimuscarinic agent: Average dry mouth burden estimate = 5.7
(p=0.02 vs. adrenergic agonist)

Average dry mouth burden experienced per day

Study day

β₃ adrenergic receptor agonist — Mild TEAE — Moderate TEAE — Severe TEAE — Antimuscarinic agent

Difference in dry mouth burden experienced each day

Between-group difference in dry mouth burden

501

Receive and store adverse event data for each day of a treatment period

502

Process adverse event data in accordance with severity indicators

503

Generate output resulting from the processing used to configure a graphical display

# FIGURE 5

Figure 6

Europäisches
Patentamt

European
Patent Office

Office européen
des brevets

# EUROPEAN SEARCH REPORT

Application Number

EP 16 07 5001

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2014/358576 A1 (HOFFMAN KEITH B [US] ET AL) 4 December 2014 (2014-12-04) * the whole document * | 1-29 | INV. G06F19/00 |
| X | US 2004/117126 A1 (FETTERMAN JEFFREY E [US] ET AL) 17 June 2004 (2004-06-17) * abstract * * figures 1,2,4,5,7-9 * * claims 1,4,43,45 * | 1-29 | |
| X | WO 2014/017969 A2 (SCIENT SWEDEN AB [SE]) 30 January 2014 (2014-01-30) * abstract * * table 12 * * figures 6,7,10 * | 1-29 | |
| X | US 2008/081959 A1 (JUNG EDWARD K Y [US] ET AL) 3 April 2008 (2008-04-03) * paragraphs [0095], [0096], [0138] * * figures 1-22 * | 1-29 | |
| X | BAKER R. A. ET AL: "Atypical antipsychotic drugs and diabetes mellitus in the US Food and Drug Administration Adverse Event database: a systematic Bayesian signal detection analysis.", PSYCHOPHARMACOLOGY BULLETIN, vol. 42, no. 1, 1 January 2009 (2009-01-01), pages 11-31, XP008180763, ISSN: 0048-5764 * abstract * * page 13, line 19 - page 16, line 32 * * tables 2A,2B,2C,3 * | 1-29 | TECHNICAL FIELDS SEARCHED (IPC)  G06F |
| X | US 2003/135128 A1 (SUFFIN STEPHEN C [US] ET AL) 17 July 2003 (2003-07-17) * paragraphs [0200], [0211] * | 1-29 | |

-/--

The present search report has been drawn up for all claims

| Place of search | Date of completion of the search | Examiner |
|---|---|---|
| The Hague | 11 July 2016 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 16 07 5001

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 8 275 552 B1 (TROTTI III ANDREA [US]) 25 September 2012 (2012-09-25) * the whole document * ----- | 1-29 | |

TECHNICAL FIELDS
SEARCHED      (IPC)

The present search report has been drawn up for all claims

| Place of search | Date of completion of the search | Examiner |
|---|---|---|
| The Hague | 11 July 2016 | Godzina, Przemyslaw |

CATEGORY OF CITED DOCUMENTS

X : particularly relevant if taken alone
Y : particularly relevant if combined with another
     document of the same category
A : technological background
O : non-written disclosure
P : intermediate document

T : theory or principle underlying the invention
E : earlier patent document, but published on, or
     after the filing date
D : document cited in the application
L : document cited for other reasons

 
& : member of the same patent family, corresponding
     document

EPO FORM 1503 03.82 (P04C01)

EP 3 125 138 A1

## ANNEX TO THE EUROPEAN SEARCH REPORT
## ON EUROPEAN PATENT APPLICATION NO.

EP 16 07 5001

This annex lists the patent family members relating to the patent documents cited in  the above-mentioned European search report.
The members are as contained in the European Patent Office EDP file on
The European Patent Office is in no way liable for these particulars which are merely given for the purpose of information.

11-07-2016

| Patent document cited in search report | | Publication date | Patent family member(s) | | Publication date |
|---|---|---|---|---|---|
| US 2014358576 | A1 | 04-12-2014 | US 2014358576 A1<br>WO 2014186608 A1 | | 04-12-2014<br>20-11-2014 |
| US 2004117126 | A1 | 17-06-2004 | NONE | | |
| WO 2014017969 | A2 | 30-01-2014 | EP 2877950 A2<br>SE 1250894 A1<br>US 2015193597 A1<br>WO 2014017969 A2 | | 03-06-2015<br>25-01-2014<br>09-07-2015<br>30-01-2014 |
| US 2008081959 | A1 | 03-04-2008 | NONE | | |
| US 2003135128 | A1 | 17-07-2003 | AT 433163 T<br>AU 2002329590 B2<br>CA 2452883 A1<br>CA 2752782 A1<br>EP 1414343 A2<br>EP 2159723 A1<br>EP 2275959 A2<br>EP 2323056 A1<br>ES 2329452 T3<br>US 2003135128 A1<br>US 2008125669 A1<br>US 2009137923 A1<br>US 2009157662 A1<br>US 2010143256 A1<br>WO 03005899 A2 | | 15-06-2009<br>19-10-2006<br>23-01-2003<br>23-01-2003<br>06-05-2004<br>03-03-2010<br>19-01-2011<br>18-05-2011<br>26-11-2009<br>17-07-2003<br>29-05-2008<br>28-05-2009<br>18-06-2009<br>10-06-2010<br>23-01-2003 |
| US 8275552 | B1 | 25-09-2012 | NONE | | |

EPO FORM P0459

For more details about this annex : see Official Journal of the European Patent Office, No. 12/82

**REFERENCES CITED IN THE DESCRIPTION**

**Non-patent literature cited in the description**

- **M. BACKONJA et al.** NGX-4010, a high-concentration capsaicin patch, for the treatment of postherpetic neuralgia: a randomized double-blind study. *Lancet Neurol.,* December 2008, vol. 7 (12), 1106-12
- **T.M. BEER et al.** Enzalutamide in Metastatic prostate cancer before chemotherapy. *N Engl J Med,* 2014, vol. 371 (5), 424-33
- **HAANPÄÄ et al.** *Eur J Pain,* 2015

- **BATISTA, J.E. ; KÖLBL, H. ; HERSCHORN, S. et al.** The efficacy and safety of mirabegron compared with solifenacin in overactive bladder patients dissatisfied with previous antimuscarinic treatment due to lack of efficacy: results of a non-inferiority, randomized, phase IIIb trial. *Ther Adv Urol,* 2015, vol. 7, 167-179